# EUROPEAN PATENT SPECIFICATION

(11) **EP 3 777 678 B1**
(45) Date of publication and mention of the grant of the patent: **26.10.2022**
(21) Application number: 19775166.2
(22) Date of filing: 26.03.2019
(51) Int. Cl.: A61B 5/107, A61B 5/00

(54) **MEASUREMENT DEVICE**
MESSVORRICHTUNG
DISPOSITIF DE MESURE

(30) Priority: 26.03.2018 JP 2018058070
(43) Date of publication of application: 17.02.2021
(73) Proprietor: TERUMO Kabushiki Kaisha, Tokyo 151-0072 (JP)
(72) Inventor: MAKI, Shin, Ashigarakami-gun, Kanagawa 259-0151 (JP); UEMURA, Tomoko, Ashigarakami-gun, Kanagawa 259-0151 (JP)
(74) Representative: Casalonga
(86) International application number: PCT/JP2019/012816
(87) International publication number: WO 2019/189155

(56) References cited:
- WO-A1-2017/121434
- WO-A1-2017/121434
- BE-B1- 1 024 423
- JP-A- H05 237 119
- JP-A- 2009 526 590
- US-A- 3 820 529
- US-A- 4 966 155
- US-A- 5 732 475
- US-A1- 2012 179 020
- US-A1- 2016 015 297

## Description

### Technical Field

The present invention relates to a measurement apparatus that can measure the amount of edema of a limb.

A measurement apparatus according to the preamble of claim 1 is already known e.g. from WO 2017/121434 A1 or US 4 966 155 A.

### Background Art

Edema of a limb is known as one of symptoms that appear in association with diseases such as heart failure and liver failure and grasping the amount of edema of a limb is important in making a diagnosis of these diseases and so forth.

For example, in Patent Document 1 to be described below, an apparatus is disclosed that can measure the amount of edema of a limb through winding an elongated band around the limb and detecting the circumferential length of the limb based on the coupling position at which the end portions of the band are coupled to each other.

### Prior Art Document

### Patent Document

Patent Document 1: Japanese Patent Laid-open No. 2002-159473

### Summary of Invention

### Technical Problem

The edema of a limb generated in association with diseases such as heart failure and liver failure is known as a pitting edema with which, when the limb is pressed, body fluid accumulated at the pressed place of the limb escapes from the pressed place and an indentation is generated at the pressed place. For this reason, when an edema generated in a limb is a pitting edema, the apparatus as in the above-described patent document 1 involves a possibility that the band bites into the limb and an indentation is generated and it is impossible to measure the accurate amount of edema.

The present invention is made in view of the above-described circumstances and intends to provide a measurement apparatus that can measure the amount of edema of a limb more accurately.

### Technical Solution

A measurement apparatus according to the present invention that achieves the above-described object is a measurement apparatus according to independent claim 1. The dependent claims relate to advantageous embodiments of the present invention.

### Advantageous Effects

According to the measurement apparatus in accordance with the present invention, by the sensor that freely expands and contracts and is capable of detecting change in an electrical characteristic in association with expansion or contraction, the amount of edema of the limb can be measured while generation of an indentation in the limb is suppressed. Therefore, according to the present invention, a measurement apparatus that can measure the amount of edema of the limb more accurately can be provided.

### Brief Description of Drawings

[FIG. 1]

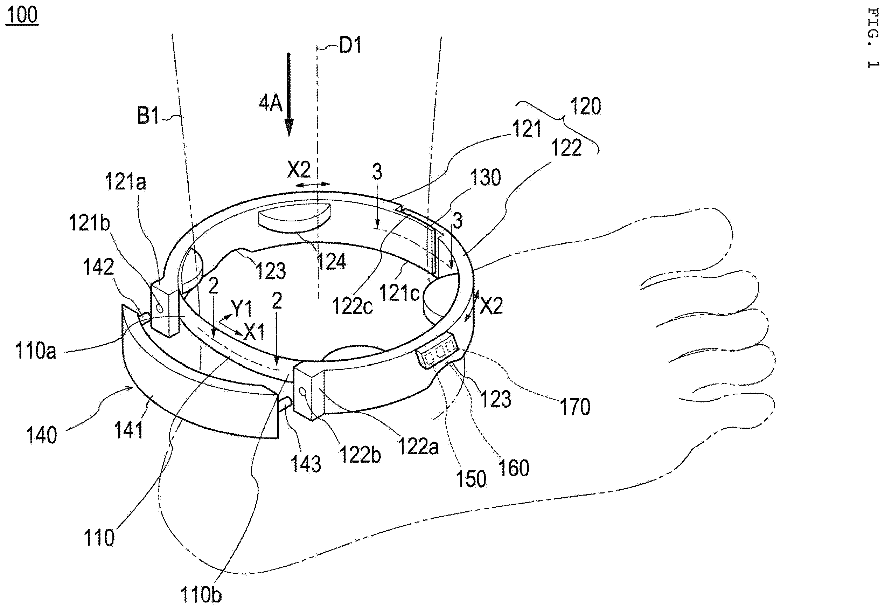

   FIG. 1 is a perspective view depicting a measurement apparatus according to a first embodiment of the present invention.
[FIG. 2]

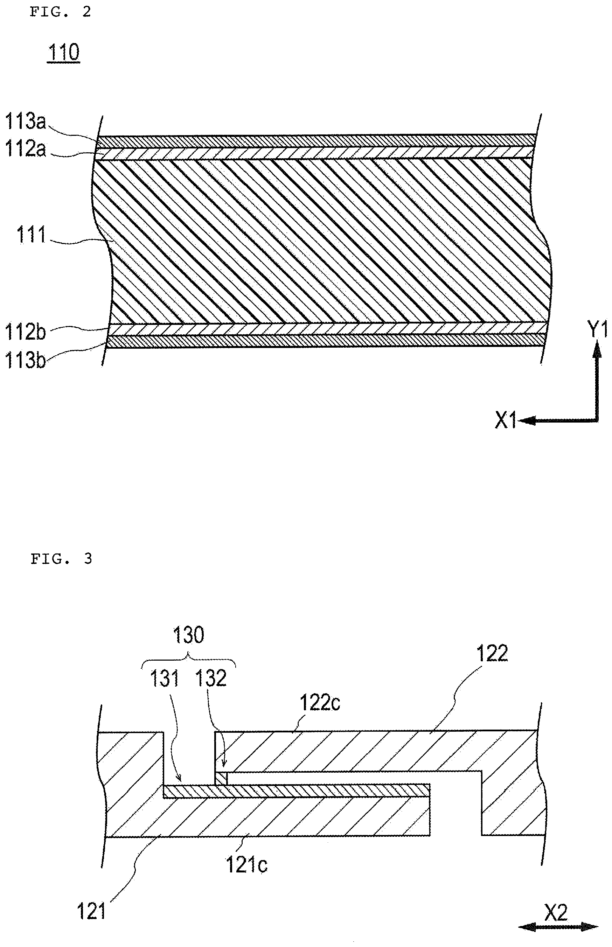

   FIG. 2 is a sectional view along line 2-2 in FIG. 1.
[FIG. 3]
   FIG. 3 is a sectional view along line 3-3 in FIG. 1.
[FIG. 4A]

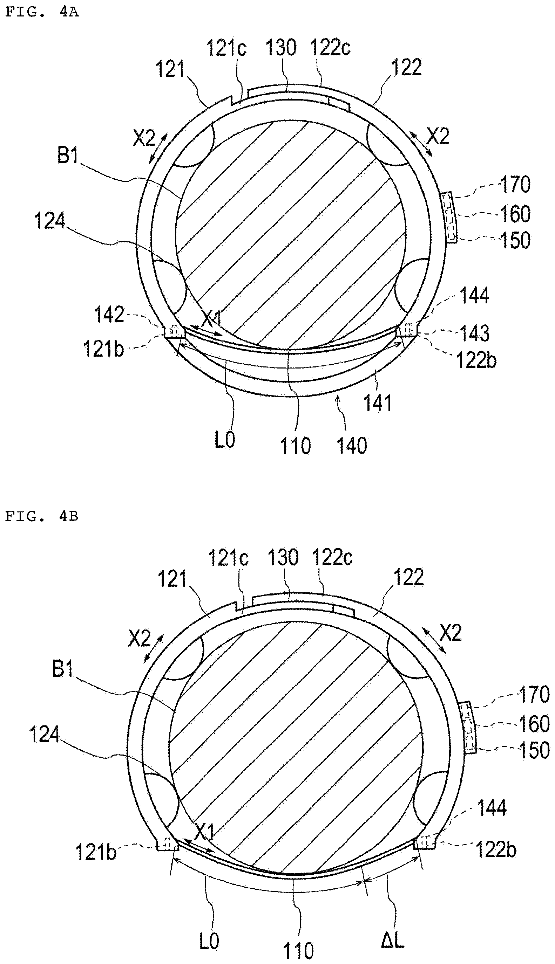

   FIG. 4A is an arrow view as viewed from a direction of an arrow 4A in FIG. 1 and is a diagram depicting a state in initial setting.
[FIG. 4B]
   FIG. 4B is an arrow view as viewed from a direction of an arrow 4A in FIG. 1 and is a diagram depicting a state in measurement.
[FIG. 5]

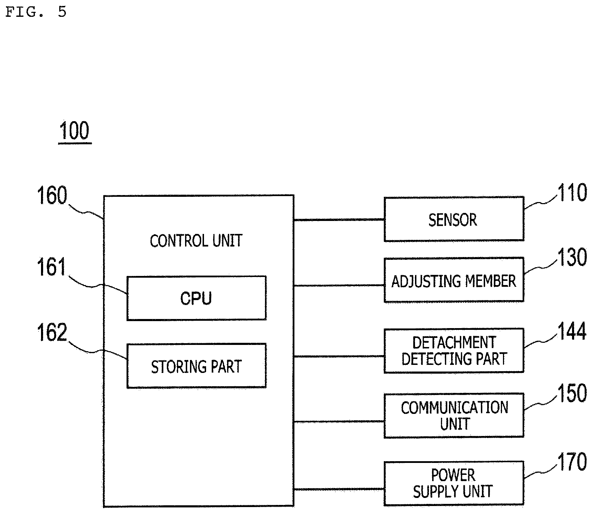

   FIG. 5 is a block diagram of the measurement apparatus according to the first embodiment of the present invention.
[FIG. 6]

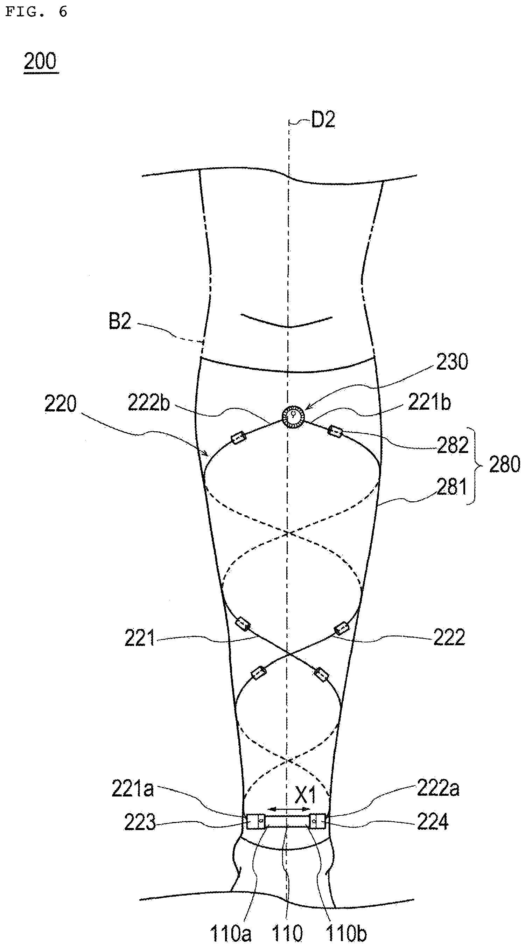

   FIG. 6 is a plan view depicting the measurement apparatus according to a second embodiment of the present invention.
[FIG. 7]

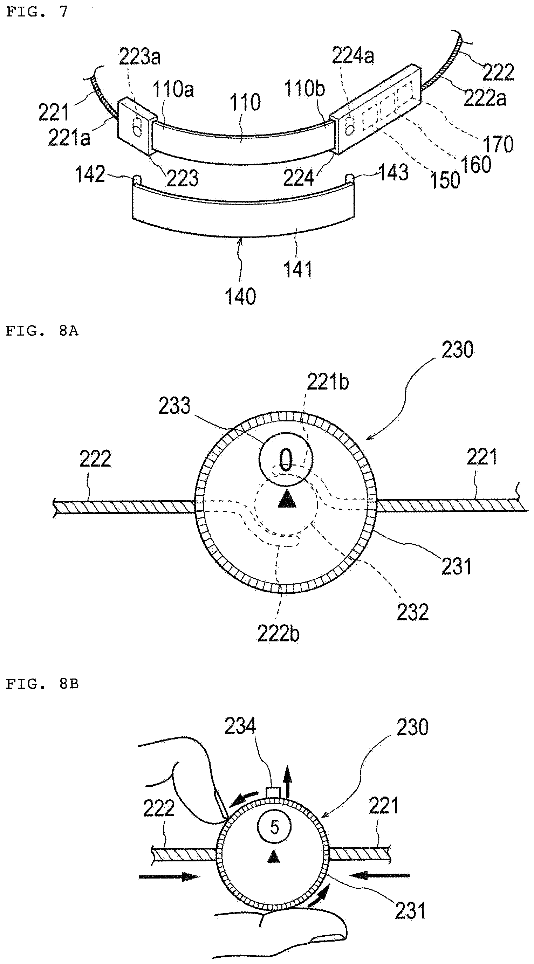

   FIG. 7 is a perspective view depicting a sensor and surroundings thereof in the measurement apparatus according to the second embodiment of the present invention.
[FIG. 8A]
   FIG. 8A is a plan view depicting an adjusting member and surroundings thereof in the measurement apparatus according to the second embodiment of the present invention.
[FIG. 8B]
   FIG. 8B is a plan view depicting the adjusting member and surroundings thereof in the measurement apparatus according to the second embodiment of the present invention.

### Modes for Carrying Out the Invention

Embodiments of the present invention will be described below with reference to the accompanying drawings. Note that, in description of the drawings, the same element is given the same symbols and overlapping description is omitted. Furthermore, the dimension ratio of the drawing is exaggerated for convenience of explanation and is different from the actual ratio in some cases.

### <First Embodiment>

FIG. 1 is a diagram for explaining the overall configuration of a measurement apparatus 100 according to a first embodiment. FIGS. 2 to 5 are diagrams for explaining the respective parts of the measurement apparatus 100 according to the first embodiment.

As depicted in FIG. 1, the measurement apparatus 100 according to the first embodiment is configured as apparatus that is mounted on an ankle B1 (corresponding to "limb") of a patient with heart failure, liver failure, or the like and can measure the amount of edema of the ankle B1 by measuring the circumferential length of the ankle B1. Note that, in the present specification, "circumferential length of the ankle B1" means the length of the ankle B1 around a long axis direction D1 of a leg. Furthermore, the person who wears the measurement apparatus 100 is not particularly limited to the patient with heart failure, liver failure, or the like.

When being described in outline, the measurement apparatus 100 according to the first embodiment has a sheet-shaped sensor 110 having electrical conductivity, a support member 120 that is attached to the sensor 110 and surrounds a limb together with the sensor 110 in a mounted state in which the measurement apparatus 100 is mounted on the ankle B1, and an adjusting member 130 that can adjust the length of the support member 120. The measurement apparatus 100 has also a restricting member 140 that can restrict the length of the sensor 110, a communication unit 150 that can communicate with external apparatus, a control unit 160 that controls operation of the respective parts, and a power supply unit 170 that can supply power to the respective parts. The respective parts of the measurement apparatus 100 will be described in detail below.

### (Sensor)

In the present embodiment, the sensor 110 has electrical conductivity and a sheet shape as depicted in FIG. 1. In the present embodiment, the sensor 110 freely expands and contracts in one direction (hereinafter, referred to as "expansion-contraction direction X1") that intersects a thickness direction Y1 of the sensor 110 and is configured to be capable of detecting change in an electrical characteristic in association with expansion or contraction in the expansion-contraction direction X1. Note that, in the present specification, "freely expands and contracts" means that the sensor 110 mounted on a limb freely gets elastically deformed in the expansion-contraction direction X1 in such a manner as to follow change in the amount of edema of the limb (volume of the limb).

In the present embodiment, the sensor 110 has an elongated sheet shape as depicted in FIG. 1. In the present embodiment, the expansion-contraction direction X1 of the sensor 110 corresponds to the longitudinal direction of the sensor 110 (circumferential direction in the mounted state in which the measurement apparatus 100 is mounted on the ankle B). However, the shape of the sensor 110 is not particularly limited as long as it is a sheet shape. For example, the sensor 110 may have a substantially square shape (that is, the sensor 110 does not need to have an elongated shape). Because having the sheet shape as above, the sensor 110 can easily expand and contract in the one direction (expansion-contraction direction X1) that intersects the thickness direction Y1. Thus, by the sensor 110, the amount of edema of the ankle B1 can be measured while generation of an indentation in the ankle B1 is suppressed.

In the present embodiment, as depicted in FIG. 2, the sensor 110 is configured by a capacitor including a base 111 having an elongated sheet shape, a pair of electrically-conductive layers 112a and 112b stacked in the thickness direction Y1 to sandwich the base 111, and a pair of protective layers 113a and 113b stacked in the thickness direction Y1 to sandwich the pair of electrically-conductive layers 112a and 112b.

The base 111 is configured to freely expand and contract in the expansion-contraction direction X1. In the present embodiment, the base 111 functions as a dielectric of the capacitor. When the base 111 expands or contracts in the expansion-contraction direction X1, the length of the base 111 in the expansion-contraction direction X1 and the length (thickness) thereof in the thickness direction Y1 change. The capacitance (corresponding to "electrical characteristic") of the sensor 110 depends on the length of the sensor 110 in the expansion-contraction direction X1 and the thickness thereof (distance between the pair of electrically-conductive layers 112a and 112b). Thus, the amount of expansion or contraction of the sensor 110 in the expansion-contraction direction X1, i.e., the amount of edema of the ankle B11, can be measured by detecting the capacitance that is an electrical characteristic of the sensor 110.

It is preferable for the base 111 to be composed of a material mainly containing an elastomer material because the elastomer material is excellent in the elasticity. As the elastomer material used for the base 111, for example, natural rubber, isoprene rubber, nitrile rubber (NBR), ethylene propylene rubber (EPDM), styrene-butadiene rubber (SBR), butadiene rubber (BR), chloroprene rubber (CR), silicone rubber, fluorine rubber, acrylic rubber, hydrogenated nitrile rubber, urethane rubber, what is obtained by combining two or more kinds from them, and so forth are cited. In particular, it is preferable to use urethane rubber as the elastomer material used for the base 111 because the urethane rubber has a small permanent set and is excellent in the adhesiveness with carbon nanotubes contained in the respective electrically-conductive layers 112a and 112b to be described later. Furthermore, the base 111 may contain, besides the elastomer material, additive agents such as dielectric filler (barium titanate or the like), plasticizer, chain extender, cross-linker, catalyst, vulcanization accelerator, antioxidant, anti-aging agent, and colorant within a range in which the elasticity is not inhibited.

The respective electrically-conductive layers 112a and 112b are integrated with the base 111 and are configured to be capable of expanding and contracting in the expansion-contraction direction X1 integrally with the base 111.

The respective electrically-conductive layers 112a and 112b are electrically connected to the control unit 160 through a transmitter (diagrammatic representation is omitted) or the like that allows AD conversion of a signal from the sensor 110.

The constituent material of the respective electrically-conductive layers 112a and 112b is not particularly limited as long as the material has electrical conductivity and can expand and contract integrally with the base 111. For example, the electrically-conductive layers 112a and 112b can be formed by a material mainly containing carbon nanotubes. The carbon nanotube used for the respective electrically-conductive layers 112a and 112b is not particularly limited. For example, single-walled carbon nanotube, multi-walled carbon nanotube, what is obtained by mixing single-walled carbon nanotube and multi-walled carbon nanotube, and so forth can be used. The respective electrically-conductive layers 112a and 112b may contain, besides the carbon nanotubes, binder, cross-linker, vulcanization accelerator, vulcanization assistant, anti-aging agent, plasticizer, softener, colorant, and so forth. As the binder used for the respective electrically-conductive layers 112a and 112b, for example, the following substances are cited although the binder is not particularly limited thereto: butyl rubber, ethylene propylene rubber, polyethylene, chlorosulfonated polyethylene, natural rubber, isoprene rubber, butadiene rubber, styrene-butadiene rubber, polystyrene, chloroprene rubber, nitrile rubber, polymethyl methacrylate, polyvinyl acetate, polyvinyl chloride, acrylic rubber, styrene-ethylene-butylene-styrene block copolymer, what is obtained by combining two or more kinds from them, and so forth.

The protective layers 113a and 113b are integrated with the respective electrically-conductive layers 112a and 112b and are configured to be capable of expanding and contracting in the expansion-contraction direction X1 integrally with the base 111 and the pair of electrically-conductive layers 112a and 112b.

As the constituent material of the protective layers 113a and 113b, a material similar to the constituent material of the base 111 can be used, for example.

Note that the sensor 110 is not particularly limited as long as it has electrical conductivity and a sheet shape and freely expands and contracts in one direction that intersects the thickness direction Y1 and is capable of detecting change in an electrical characteristic in association with expansion or contraction. For example, the sensor 110 may be configured with the base 111 and one electrically-conductive layer 112a disposed on one surface of the base 111. In this case, the distance between the carbon nanotubes in the electrically-conductive layer 112a changes in association with expansion or contraction of the sensor 110 and thereby the electrical resistance of the sensor 110 changes. Thus, change in the amount of expansion or contraction of the sensor 110 in the expansion-contraction direction X1, i.e., change in the amount of edema of the ankle B11, can be measured by detecting the electrical resistance that is an electrical characteristic of the sensor 110.

### (Support Member)

In the present embodiment, as depicted in FIG. 1, the support member 120 is attached to both end portions 110a and 110b in the expansion-contraction direction X1 in the sensor 110 and surrounds the ankle B1 together with the sensor 110 in the mounted state in which the measurement apparatus 100 is mounted on the ankle B1. Thus, the support member 120 fixes the sensor 110 to the ankle B1 in the mounted state. In addition, when an edema is generated in the ankle B1, the support member 120 pulls both end portions 110a and 110b in the expansion-contraction direction X1 in the sensor 110 and expands the sensor 110.

In the present embodiment, the support member 120 is composed of a first belt member 121 and a second belt member 122 that have an elongated shape as depicted in FIG. 1.

One end portion 121a in a longitudinal direction X2 (circumferential direction in the mounted state) in the first belt member 121 is connected to one end portion 110a in the expansion-contraction direction X1 in the sensor 110. One end portion 122a in the longitudinal direction X2 in the second belt member 122 is connected to the other end portion 110b in the expansion-contraction direction X1 in the sensor 110. Note that, in the present specification, "end portion in the expansion-contraction direction X1 or the longitudinal direction X2" includes not only the most distal of each member in the expansion-contraction direction X1 or the longitudinal direction X2 but also a certain range from the most distal in the expansion-contraction direction X1 or the longitudinal direction X2.

In the one end portion 121a in the longitudinal direction X2 in the first belt member 121 and the one end portion 122a in the longitudinal direction X2 in the second belt member 122, hole parts 121b and 122b in which protrusions 142 and 143 of the restricting member 140 to be described later can be inserted are made.

As depicted in FIG. 4A and FIG. 4B, inside the hole part 122b, a detachment detecting part 144 that electrically detects that the protrusion 143 is not inserted in the hole part 122b (that is, the restricting member 140 to be described later has been detached) is disposed. The detachment detecting part 144 is electrically connected to the control unit 160. Note that the detachment detecting part 144 may be disposed inside the hole part 121b.

As depicted in FIG. 1, the adjusting member 130 is disposed on the other end portion 121c in the longitudinal direction X2 in the first belt member 121 and the other end portion 122c in the longitudinal direction X2 in the second belt member 122. As depicted in FIG. 4A and FIG. 4B, the measurement apparatus 100 is mounted on the ankle B1 by winding the sensor 110 and the support member 120 around the ankle B1 and interlocking the other end portion 121c in the longitudinal direction in the first belt member 121 and the other end portion 122c in the longitudinal direction in the second belt member 122 by the adjusting member 130. The first belt member 121 and the second belt member 122 are annularly disposed around the ankle B1 together with the sensor 110 in the mounted state. Thus, the measurement apparatus 100 can measure the circumferential length of the ankle B1, i.e., the amount of edema of the ankle B1.

In the first belt member 121 and the second belt member 122, as depicted in FIG. 1, a pair of recess parts 123 that hollow in the width direction of the support member 120 (long axis direction D1 of a leg in the mounted state) are made at positions opposed in the mounted state. In the mounted state, shift of the measurement apparatus 100 toward the lower side of the long axis direction D1 of the leg can be suppressed by making the pair of recess parts 123 abut against the malleoli of the ankle B1 (corresponding to projecting parts of a limb).

It is preferable to form the first belt member 121 and the second belt member 122 by a material with a higher modulus of elasticity than the sensor 110. By forming the first belt member 121 and the second belt member 122 by such a material, when an edema is generated in the ankle B1, expansion and contraction of the support member 120 are suppressed and the amount of edema of the ankle B1 can be allowed to correspond to the amount of expansion or contraction of the sensor 110 in the expansion-contraction direction X1.

Furthermore, it is preferable for the first belt member 121 and the second belt member 122 to have flexibility so as to bend according to change in the amount of edema of the ankle B1 as depicted in FIG. 4A and FIG. 4B. As the material that has a higher modulus of elasticity than the sensor 110 and has flexibility, for example, the following substances are cited although the material is not particularly limited thereto: polyvinyl chloride, polyolefin such as polyethylene, polypropylene, polybutadiene, or ethylene-vinyl acetate copolymer (EVA), polyester such as polyethylene terephthalate (PET) or polybutylene terephthalate (PBT), polyvinylidene chloride, or what is obtained by optionally combining them (blend resin, polymer alloy, layer-stacked body, or the like).

Plural protrusions 124 that protrude toward the ankle B1 are disposed on the surfaces that face the ankle B1 in the first belt member 121 and the second belt member 122. As depicted in FIG. 4A and 4B, the protrusions 124 suppress contact of the support member 120 with the ankle B1 in the mounted state. For this reason, compared with the case in which the protrusions 124 are not disposed on the support member 120 and the support member 120 gets contact with the ankle B1, the measurement apparatus 100 according to the present embodiment can reduce the area of generation of an indentation even when the indentation is generated.

It is preferable that the end portion on the side of contact with the ankle B1 in each protrusion 124 have a rounded shape in order to suppress feeling of pain by the wearing person (patient). The plural protrusions 124 are disposed to be separate from each other and line up in the longitudinal direction X2 of the first belt member 121 and the second belt member 122. Note that the number of protrusions 124 included in the measurement apparatus 100 is not particularly limited although the form in which the measurement apparatus 100 includes four protrusions 124 is depicted in FIG. 4A. Furthermore, the measurement apparatus 100 does not need to include the protrusions 124.

It is preferable to form each protrusion 124 by a material having hardness at such a degree that the protrusion 124 does not get deformed when being pressed against the ankle B1 in supporting the support member 120 with respect to the ankle B1. As such a material, for example, the following substances are cited although the material is not particularly limited thereto: acrylic resin, polyvinyl chloride (particularly unplasticized polyvinyl chloride), polyolefin such as polyethylene, polypropylene, and polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyester such as polyethylene terephthalate (PET) and polybutylene terephthalate (PBT), butadiene-styrene copolymer, aromatic or aliphatic polyamide, fluorine-based resin such as polytetrafluoroethylene, and so forth.

Note that the configuration of the support member 120 is not limited to the above description as long as the support member 120 is attached to both end portions 110a and 110b in the expansion-contraction direction X1 in the sensor 110 and can surround the ankle B1 together with the sensor 110. For example, the support member 120 may be formed of one elongated belt member. Furthermore, the support member 120 may be formed of one or two elongated wire members.

### (Adjusting Member)

As depicted in FIG. 4A, the adjusting member 130 is configured to be capable of adjusting the length (circumferential length) of the part that surrounds the ankle B1 (hereinafter, referred to as "surrounding part") in the support member 120 in the mounted state.

In the present embodiment, as depicted in FIG. 3, the adjusting member 130 includes a first adjusting part 131 disposed on the other end portion 121c in the longitudinal direction X2 in the first belt member 121 and a second adjusting part 132 disposed on the other end portion 122c in the longitudinal direction X2 in the second belt member 122.

The first adjusting part 131 and the second adjusting part 132 are configured to be capable of interlocking and separating the other end portion 121c in the longitudinal direction X2 in the first belt member 121 and the other end portion 122c in the longitudinal direction X2 in the second belt member 122. In addition, the first adjusting part 131 and the second adjusting part 132 have a function as an interlocking part that can adjust the interlocking position in the longitudinal direction X2. Although being not particularly limited, for example, the interlocking part may be configured to mechanically couple the other end portion 121c in the longitudinal direction X2 in the first belt member 121 and the other end portion 122c in the longitudinal direction X2 in the second belt member 122 through fitting or the like, or may be configured to couple the other end portion 121c in the longitudinal direction X2 in the first belt member 121 and the other end portion 122c in the longitudinal direction X2 in the second belt member 122 by an attraction force such as a magnetic force.

The first adjusting part 131 and the second adjusting part 132 have also a function as a length detecting part that can electrically detect the circumferential length of the surrounding part of the support member 120. In the present embodiment, the first adjusting part 131 and the second adjusting part 132 are configured to be capable of electrically detecting the circumferential length of the surrounding part by electrically detecting the contact position of the first adjusting part 131 and the second adjusting part 132 in the longitudinal direction X2.

It is preferable that the contact position of the first adjusting part 131 and the second adjusting part 132 can be detected continuously in the longitudinal direction X2. As a method for detecting the contact position continuously in the longitudinal direction X2, for example, a method is cited in which the first adjusting part 131 is configured by an elongated resistive element that extends in the longitudinal direction X2 and the second adjusting part 132 is configured by a terminal that can get contact with an optional position in the longitudinal direction X2 on the resistive element, although the method is not particularly limited thereto. One end portion in the longitudinal direction X2 in the resistive element and the terminal are electrically connected to the control unit 160. Because the electrical resistance changes according to the contact position of the resistive element and the terminal in the longitudinal direction X2, the contact position of the first adjusting part 131 and the second adjusting part 132 in the longitudinal direction X2 can be electrically detected. Note that the first adjusting part 131 may be configured by the terminal and the second adjusting part 132 may be configured by the resistive element.

The interlocking position of the first adjusting part 131 and the second adjusting part 132 may be allowed to be adjusted and detected discretely in the longitudinal direction X2. As a method for adjusting and detecting the interlocking position discretely in the longitudinal direction X2 by the first adjusting part 131 and the second adjusting part 132, for example, a method in which the first adjusting part 131 is configured by plural electrical contacts disposed to line up in the longitudinal direction X2 and the second adjusting part 132 is configured by a terminal that can get contact with each electrical contact, and so forth, are cited although the method is not particularly limited thereto. Each electrical contact and the terminal are electrically connected to the control unit 160. The interlocking position of the first adjusting part 131 and the second adjusting part 132 in the longitudinal direction X2 can be detected according to which electrical contact is energized. Note that the first adjusting part 131 may be configured by the terminal and the second adjusting part 132 may be configured by the plural electrical contacts. In either case, it is preferable to set the distance between the electrical contacts adjacent to each other to such a degree that the measurement apparatus 100 is allowed to fit an ankle of a wearing person irrespective of the individual difference of the wearing person.

Note that the position at which the adjusting member 130 is disposed is not limited to the above description as long as the circumferential length of the surrounding part of the support member 120 can be adjusted. For example, the adjusting member 130 may be disposed at an intermediate part in the longitudinal direction X2 in the first belt member 121 and the second belt member 122.

### (Restricting Member)

As depicted in FIG. 4A and FIG. 4B, the restricting member 140 is configured to be attachable to the support member 120 and be detachable. As depicted in FIG. 4A, in the state in which the restricting member 140 is attached to the support member 120, the restricting member 140 covers the sensor 110 in the expansion-contraction direction X1 and restricts expansion and contraction of the sensor 110 in the expansion-contraction direction X1. Thus, the measurement apparatus 100 can adjust the circumferential length of the surrounding part of the support member 120 by the adjusting member 130 in the state in which the length of the sensor 110 in the expansion-contraction direction X1 is kept at a reference length L0 by the restricting member 140. Thus, as depicted in FIG. 4B, the measurement apparatus 100 can measure an amount ΔL of expansion or contraction in the expansion-contraction direction X1 from the reference length L0 of the sensor 110 in the state in which the restricting member 140 has been detached from the support member 120. As above, the restricting member 140 and the adjusting member 130 are used at the time of initial setting in which the length of the surrounding part of the adjusting member 130 is adjusted in the state in which the length of the sensor 110 in the expansion-contraction direction X1 is kept at the reference length L0, before measurement by the measurement apparatus 100 is started.

As depicted in FIG. 1, the restricting member 140 includes a main body part 141 that covers the sensor 110 in the expansion-contraction direction X1 in the state of being attached to the support member 120 and the two protrusions 142 and 143 that are disposed at both end portions of the main body part 141 and can be inserted in the hole parts 121b and 122b of the support member 120.

The main body part 141 has a circular arc shape in the present embodiment. However, the shape of the main body part 141 is not particularly limited as long as the main body part 141 can cover the sensor 110 in the expansion-contraction direction X1 in the state of being attached to the support member 120.

It is preferable to form the restricting member 140 by a material with higher hardness than the sensor 110 in order to restrict expansion and contraction of the sensor 110 in the state in which the restricting member 140 is attached to the support member 120. As such a material, for example, the following substances are cited although the material is not particularly limited thereto: acrylic resin, polyvinyl chloride (particularly unplasticized polyvinyl chloride), polyolefin such as polyethylene, polypropylene, and polybutadiene, polystyrene, poly-(4-methylpentene-1), polycarbonate, ABS resin, polymethyl methacrylate (PMMA), polyacetal, polyacrylate, polyacrylonitrile, polyvinylidene fluoride, ionomer, acrylonitrile-butadiene-styrene copolymer, polyester such as polyethylene terephthalate (PET) and polybutylene terephthalate (PBT), butadiene-styrene copolymer, aromatic or aliphatic polyamide, fluorine-based resin such as polytetrafluoroethylene, and so forth.

Note that the configuration of the restricting member 140 is not particularly limited as long as the restricting member 140 is attachable to the support member 120 and is detachable and, in the state of being attached to the support member 120, covers the sensor 110 in the expansion-contraction direction X1 and can restrict expansion and contraction of the sensor 110 in the expansion-contraction direction X1. For example, the restricting member 140 may be configured to be attachable and detachable to and from the support member 120 by an attraction force such as a magnetic force instead of being attached to the support member 120 through insertion of the protrusions 142 and 143 in the hole parts 121b and 122b of the support member 120.

### (Communication Unit)

The communication unit 150 is an interface for transmitting measurement data to external apparatus (diagrammatic representation is omitted) or the like. The external apparatus is not particularly limited and is a PC or the like of a doctor or the like, for example.

### (Control Unit)

As depicted in FIG. 5, the control unit 160 is configured by a publicly-known microcomputer including a CPU 161, a storing part 162, and so forth. The CPU 161 executes control of the respective parts, various kinds of arithmetic processing, and so forth in accordance with various programs stored in the storing part 162. The storing part 162 is configured by a ROM (Read Only Memory) that stores various programs and various kinds of data, a RAM (Random Access Memory) that temporarily stores program and data as a work area, and so forth.

The control unit 160 is electrically connected to the sensor 110, the adjusting member 130, the detachment detecting part 144, the communication unit 150, and the power supply unit 170 and controls operation of them.

The CPU 161 controls detection operation of the capacitance of the sensor 110. The CPU 161 converts the detected capacitance of the sensor 110 to the amount ΔL of expansion or contraction of the sensor 110 in the expansion-contraction direction X1. The CPU 161 causes the storing part 162 to store the calculated amount ΔL of expansion or contraction of the sensor 110 in the expansion-contraction direction X1.

The CPU 161 controls detection operation of the interlocking position of the first adjusting part 131 and the second adjusting part 132 of the adjusting member 130 in the longitudinal direction X2 and calculates the circumferential length of the ankle B1 (hereinafter, referred to as "initial circumferential length of the ankle B1") from the detected interlocking position of the first adjusting part 131 and the second adjusting part 132 in the longitudinal direction X2. The CPU 161 causes the storing part 162 to store the calculated initial circumferential length of the ankle B1.

The control unit 160 controls transmission operation in which the communication unit 150 transmits measurement data to external apparatus.

### (Power Supply Unit)

The power supply unit 170 is not particularly limited as long as it can supply power to the respective parts of the measurement apparatus 100, and can be configured by a primary battery, secondary battery, or the like.

In the present embodiment, as depicted in FIG. 1, the communication unit 150, the control unit 160, and the power supply unit 170 are disposed on the surface (outer surface in the mounted state) on the opposite side to the surface that faces the ankle B1 (inner surface in the mounted state) in the second belt member 122. However, the position at which the communication unit 150, the control unit 160, and the power supply unit 170 are disposed is not particularly limited as long as expansion and contraction of the sensor 110 are not inhibited. For example, the communication unit 150, the control unit 160, and the power supply unit 170 may be disposed on the outer surface of the first belt member 121 or may be disposed to be incorporated in the first belt member 121 or the second belt member 122.

### (Use Method)

Next, a use method of the measurement apparatus 100 according to the present embodiment will be described.

First, initial setting is carried out before the measurement apparatus 100 starts measurement.

In the initial setting, first, the wearing person attaches the restricting member 140 to the support member 120 as depicted in FIG. 4. Thereby, the length of the sensor 110 in the expansion-contraction direction X1 is kept at the reference length L0.

Next, the wearing person winds the sensor 110 and the support member 120 around the ankle B1 and couples the other end portion 121c in the longitudinal direction X2 in the first belt member 121 and the other end portion 122c in the longitudinal direction X2 in the second belt member 122 by the adjusting member 130. Thereby, the measurement apparatus 100 is mounted on the ankle B1. Furthermore, at this time, the wearing person adjusts the interlocking position of the first adjusting part 131 and the second adjusting part 132 of the adjusting member 130 in the longitudinal direction X2 so that the measurement apparatus 100 may fit the ankle B1.

Next, the wearing person detaches the restricting member 140 from the support member 120. Due to this, the detachment detecting part 144 detects that the restricting member 140 has been detached from the support member 120. Along with this, the CPU 161 of the control unit 160 starts measurement of the amount ΔL of expansion or contraction of the sensor 110. Furthermore, along with this, the adjusting member 130 detects the interlocking position of the first adjusting part 131 and the second adjusting part 132 in the longitudinal direction X2. The CPU 161 calculates the initial circumferential length of the ankle B1 based on the detected interlocking position of the first adjusting part 131 and the second adjusting part 132 in the longitudinal direction X2. The CPU 161 causes the storing part 162 to store the calculated initial circumferential length of the ankle B1.

In the present embodiment, the CPU 161 is configured to cause the sensor 110 to detect the capacitance at a predetermined time interval (for example, every minute to every hour). The CPU 161 converts the detected capacitance of the sensor 110 to the amount of expansion or contraction of the sensor 110 in the expansion-contraction direction X1. The CPU 161 causes the storing part 162 to store data of the calculated amount ΔL of expansion or contraction of the sensor 110 in the expansion-contraction direction X1.

Next, at a predetermined timing, the control unit 160 transmits the initial circumferential length of the ankle B1 and the measured amount ΔL of expansion or contraction of the sensor 110 to external apparatus through the communication unit 150. The timing at which the control unit 160 transmits the amount ΔL of expansion or contraction of the sensor 110 to the external apparatus is not particularly limited. For example, the timing when the used volume of the storing part 162 exceeds a threshold, a predetermined time internal (for example, every other day), and so forth are cited.

As above, the measurement apparatus 100 according to the above-described embodiment is the measurement apparatus that can measure the amount of edema of the ankle B1. The measurement apparatus 100 has the sensor 110 that has a sheet shape and freely expands and contracts in one direction (expansion-contraction direction X1) that intersects the thickness direction Y1 and is capable of detecting change in an electrical characteristic (capacitance) in association with expansion or contraction, and the support member 120 that is attached to both end portions 110a and 110b in the expansion-contraction direction X1 in the sensor 110 and surrounds the ankle B1 together with the sensor 110 in the mounted state in which the measurement apparatus 100 is mounted on the ankle B1.

According to the above-described measurement apparatus 100, by the sensor 110 that freely expands and contracts and is capable of detecting change in an electrical characteristic in association with expansion or contraction, the amount of edema of the ankle B1 can be measured while generation of an indentation in the ankle B1 is suppressed. Therefore, the measurement apparatus 100 that can measure the amount of edema of the ankle B1 more accurately can be provided.

Furthermore, the sensor 110 includes the base 111 containing an elastomer material and the electrically-conductive layers 112a and 112b that are stacked in the thickness direction Y1 of the base 111 and freely expand and contract integrally with the base 111. Therefore, the base 111 and the electrically-conductive layers 112a and 112b expand or contract according to the amount of edema and can change the electrical characteristic of the sensor 110 in association with the expansion or contraction.

Furthermore, the support member 120 and the sensor 110 are annularly disposed around the ankle B1 in the mounted state. Therefore, the measurement apparatus 100 can measure the amount of edema of the ankle B1 by measuring change in the circumferential length of the ankle B1.

Moreover, the measurement apparatus 100 further has the adjusting member 130 that is disposed on the support member 120 and can adjust the length of the part that surrounds the ankle B1 in the support member 120 in the mounted state, and the restricting member 140 that is attachable and detachable to and from the support member 120 and restricts expansion and contraction of the sensor 110 in the state of being attached to the support member 120. Therefore, the length of the part that surrounds the ankle B1 in the support member 120 can be adjusted by the adjusting member 130 in the state in which the restricting member 140 is attached to the support member 120 and the length of the sensor 110 in the expansion-contraction direction X1 is kept at the reference length L0. Thus, the measurement apparatus 100 can measure the amount ΔL of expansion or contraction from the reference length L0 of the sensor 110.

Furthermore, the adjusting member 130 has the function as the length detecting part that can electrically detect the length of the part that surrounds the ankle B1 in the mounted state in the support member 120. Therefore, the measurement apparatus 100 can measure the circumferential length of the ankle B1.

Moreover, the plural protrusions 124 that protrude toward the ankle B1 are disposed on the surface that faces the ankle B1 in the support member 120 in the mounted state. Therefore, contact of the support member 120 with the ankle B1 can be suppressed and the area of generation of an indentation can be reduced.

Furthermore, the support member 120 is composed of a material with a higher modulus of elasticity than the sensor 110. Therefore, expansion and contraction of the support member 120 according to the amount of edema of the ankle B1 can be suppressed and the amount of edema of the ankle B1 can be associated with the amount of expansion or contraction of the sensor 110.

Moreover, in the support member 120, the recess parts 123 that are formed to hollow in the long axis direction D1 of a leg in the mounted state and abut against the malleoli of the ankle B1 are made. Therefore, in the mounted state, shift of the measurement apparatus 100 toward the lower side of the long axis direction D1 of the leg can be suppressed by making the recess parts 123 abut against the malleoli of the ankle B1.

### <Second Embodiment>

FIG. 6 is a diagram for explaining the overall configuration of the measurement apparatus 200 according to a second embodiment. FIGS. 7 to 8B are diagrams for explaining the respective parts of the measurement apparatus 200 according to the second embodiment.

As depicted in FIG. 6, the measurement apparatus 200 according to the second embodiment is different from the measurement apparatus 100 according to the first embodiment in that a support member 220 is disposed in a spiral manner around a lower leg B2 (corresponding to "limb") in the mounted state. The measurement apparatus 200 according to the second embodiment will be described below. Note that a configuration similar to that in the measurement apparatus 100 according to the first embodiment is given the same symbols and detailed description thereof is omitted.

As depicted in FIG. 6, the measurement apparatus 200 according to the second embodiment includes the sensor 110, a support member 220 that is attached to the sensor 110 and surrounds the lower leg B2 together with the sensor 110 in the mounted state, a holding member 280 that holds the support member 220, and an adjusting member 230 attached to the support member 220. As depicted in FIG. 7, the measurement apparatus 200 further has the restricting member 140 that is attachable and detachable to and from the support member 220, the communication unit 150 that can communicate with external apparatus, the control unit 160 that controls operation of the respective parts, and the power supply unit 170 that can supply power to the respective parts.

### (Support Member)

As depicted in FIG. 6, in the present embodiment, the support member 220 includes a first wire member 221 and a second wire member 222 that have an elongated shape and a first connecting part 223 and a second connecting part 224 that connect the first wire member 221 and the second wire member 222 to the sensor 110.

One end portion 221a in the longitudinal direction in the first wire member 221 is connected to the one end portion 110a in the expansion-contraction direction X1 in the sensor 110 through the first connecting part 223. One end portion 222a in the longitudinal direction in the second wire member 222 is connected to the other end portion 110b in the expansion-contraction direction X1 in the sensor 110 through the second connecting part 224.

The other end portion 221b in the longitudinal direction in the first wire member 221 and the other end portion 222b in the longitudinal direction in the second wire member 222 are connected to the adjusting member 230 as depicted in FIG. 8A.

The first wire member 221 and the second wire member 222 are disposed in a spiral manner around the lower leg B2 in the mounted state as depicted in FIG. 6. When an edema is generated in the lower leg B2, the first wire member 221 and the second wire pull the sensor 110 in the expansion-contraction direction X1. Thus, the sensor 110 can measure the amount of edema in a certain range in a long axis direction D2 of the lower leg B2 (range surrounded by the support member 220). Therefore, the measurement apparatus 200 according to the second embodiment can measure the amount of edema in a wide range compared with the measurement apparatus 100 according to the first embodiment.

It is preferable to form the first wire member 221 and the second wire member 222 by a material with a higher modulus of elasticity than the sensor 110. By forming the first wire member 221 and the second wire member 222 by such a material, when an edema is generated in the lower leg B2, expansion and contraction of the first wire member 221 and the second wire member 222 are suppressed and the amount of edema of the ankle B1 can be allowed to correspond to the amount of expansion or contraction of the sensor 110.

Furthermore, it is preferable for the first wire member 221 and the second wire member 222 to have flexibility so as to bend according to change in the amount of edema of the lower leg B2. As the material that has a higher modulus of elasticity than the sensor 110 and has flexibility, for example, what is similar to the constituent material of the support member 120 according to the first embodiment can be used although the material is not particularly limited thereto.

The first connecting part 223 and the second connecting part 224 have a plate shape as depicted in FIG. 7. In the surfaces on the opposite side to the surfaces that face the lower leg B2 in the mounted state in the first connecting part 223 and the second connecting part 224, hole parts 223a and 224a in which the protrusions 142 and 143 of the restricting member 140 can be inserted are made.

In the present embodiment, as depicted in FIG. 7, the communication unit 150, the control unit 160, the power supply unit 170, and so forth are incorporated in the second connecting part 224. However, the communication unit 150, the control unit 160, the power supply unit 170, and so forth may be incorporated in the first connecting part 223 or may be incorporated in another member of the measurement apparatus 200.

### (Holding Member)

As depicted in FIG. 6, the holding member 280 includes a tubular main body part 281 and plural insertion parts 282 that are disposed on the main body part 281 and in which the first wire member 221 and the second wire member 222 can be inserted.

The main body part 281 is configured by stockings formed by braiding strings. Therefore, the main body part 281 can get deformed to fit the lower leg B2 in the mounted state. The string to configure the main body part 281 is not particularly limited. For example, the main body part 281 can be configured by a material such as a nylon string or polyurethane, for example.

In the present embodiment, the main body part 281 is disposed to cover a region from a height below the knee to a height above the malleoli in the lower leg B2 (particularly calf) in the mounted state. Thus, the main body part 281 fits the calf and positional shift in the long axis direction D2 of the lower leg B2 can be suppressed. However, the shape of the main body part 281 is not limited to the above description. For example, the main body part 281 may be disposed to cover part of the calf in the mounted state. Furthermore, for example, the main body part 281 may have a shape that surrounds not only the lower leg B2 but also the foot tip (toe). In either case, the wearing person can easily wear the measurement apparatus 200 by making the lower leg B2 pass through the main body part 281.

In the present embodiment, each insertion part 282 has an annular shape having a hole part. The first wire member 221 and the second wire member 222 are held by the main body part 281 by being inserted through the hole parts of the insertion parts 282. The plural insertion parts 282 are disposed to line up in a spiral manner in the main body part 281 so that the first wire member 221 and the second wire member 222 may be disposed in a spiral manner around the lower leg B2 in the mounted state. However, the configuration of the insertion parts 282 is not particularly limited as long as the first wire member 221 and the second wire member 222 can be inserted therein. For example, the insertion parts 282 may be configured by hole parts made in the main body part 281.

### (Adjusting Member)

As depicted in FIG. 6, the adjusting member 230 is configured to be capable of adjusting the length of the part that surrounds the lower leg B2 in the first wire member 221 and the second wire member 222 (length of the spiral of the first wire member 221 and the second wire) in the mounted state. Thus, in the state in which the sensor 110 is kept at the reference length L0 by the restricting member 140, the length of the spiral of the first wire member 221 and the second wire member can be adjusted and the measurement apparatus 200 can be allowed to fit the lower leg B2. Therefore, the measurement apparatus 200 can measure the amount ΔL of expansion or contraction of the sensor 110 from the reference length L0.

As depicted in FIG. 8A and FIG. 8B, in the present embodiment, the adjusting member 230 includes a dial 231 that can be rotated by the wearing person with hand fingers and a rolling-up part 232 that rotates in conjunction with the rotation of the dial 231 and can roll up the first wire member 221 and the second wire member 222. The adjusting member 230 includes also a display part 233 that displays the amount of rolling-up of the first wire member 221 and the second wire member 222 and an informing part 234 that informs that the tension of the first wire member 221 and the second wire member 222 has reached a threshold.

The dial 231 has a circular cylindrical shape in the present embodiment. However, the shape of the dial 231 is not particularly limited as long as it can be rotated by the wearing person with hand fingers.

The rolling-up part 232 has a substantially circular column shape in the present embodiment. The rolling-up part 232 is disposed at substantially the center in the dial 231. In addition, the rolling-up part 232 is connected to the dial 231 and can rotate in conjunction with rotation of the dial 231. The other end portion 221b of the first wire member 221 and the other end portion 222b of the second wire member 222 are connected to the rolling-up part 232 as depicted in FIG. 8A. Thus, as depicted in FIG. 8B, in association with the rotation of the rolling-up part 232, the length of the part that surrounds the lower leg B2 in the first wire member 221 and the second wire member 222 (length of the first wire member 221 and the second wire member 222 along the spiral direction) changes.

The display part 233 is not particularly limited as long as it can display the amount of rolling-up of the first wire member 221 and the second wire member 222. For example, the display part 233 may be configured by a display that can display the amount of rotation of the rolling-up part 232 detected by a rotational angle sensor or the like. Furthermore, for example, the display part 233 may be configured by a scale that indicates the amount of rotation of the rolling-up part 232. Because the adjusting member 230 includes such a display part 233, when temporarily loosing the first wire member 221 and the second wire member 222 in detaching the measurement apparatus 100 from the lower leg B2 and wearing the measurement apparatus 100 again, the wearing person can carry out adjustment again to cause the length of the spiral of the first wire member 221 and the second wire member 222 to become the original length.

In the present embodiment, as depicted in FIG. 8B, the informing part 234 is configured by a protrusion that protrudes from the dial 231 when the tension of the first wire member 221 and the second wire member 222 reaches the threshold. However, the configuration of the informing part 234 is not particularly limited as long as the informing part 234 can inform that the tension of the first wire member 221 and the second wire member 222 has reached the threshold. For example, the informing part 234 may be configured by a display that displays information indicating that the tension of the first wire member 221 and the second wire member 222 has reached the threshold. Furthermore, the display part 233 may be configured by a speaker that sounds a buzzer when the tension of the first wire member 221 and the second wire member 222 has reached the threshold. Therefore, the wearing person can allow the tension of the first wire member 221 and the second wire member 222 to become predetermined magnitude and allow the measurement apparatus 200 to easily fit the lower leg B2.

According to the measurement apparatus 200 in accordance with the above-described second embodiment, the support member 220 is disposed in a spiral manner around the lower leg B2 in the mounted state. Therefore, the measurement apparatus 200 can measure the amount of edema in a certain range in the long axis direction D2 of the lower leg B2.

Although the present invention is described above through the embodiments, the present invention is not limited to only the respective configurations described and can be changed as appropriate based on the description of the scope of claims.

For example, the measurement apparatus according to the present invention can be applied to not only the measurement apparatus mounted on an ankle or lower leg but also the measurement apparatus mounted on a wrist, arm, or the like. In the case of mounting the measurement apparatus on a wrist, styloid processes of the wrist correspond to projecting parts of a limb.

### Description of Reference Symbols

100 Measurement apparatus
110 Sensor
111 Base
112a, 112b Electrically-conductive layer
120, 220 Support member
123 Protrusion
124 Recess part
130, 230 Adjusting member (length detecting part)
140 Restricting member
X1 Expansion-contraction direction of the sensor
Y1 Thickness direction of the sensor
X2 Longitudinal direction of the support member
D1 Long axis direction of a leg

## Claims

1. A measurement apparatus (100) capable of measuring an amount of edema of a limb, the measurement apparatus (100) comprising:
a sensor (110) that has a sheet shape and freely expands and contracts in one direction that intersects a thickness direction (Y1) and is capable of detecting change in an electrical characteristic in association with expansion or contraction; and
a support member that is attached to both end portions in an expansion-contraction direction (X1) of the sensor (110) and surrounds the limb together with the sensor (110) in a mounted state in which the measurement apparatus (100) is mounted on the limb,
**characterized in that**
said measurement apparatus (100) further comprises:
an adjusting member (130, 230) that is disposed on the support member (120, 220) and is capable of adjusting length of a part that surrounds the limb in the support member (120, 220) in the mounted state; and
a restricting member (140) that is attachable to the support member (120, 220) and is detachable from the support member (120, 220) and restricts expansion and contraction of the sensor (X1) in a state of being attached to the support member (120, 220).

2. The measurement apparatus (100) according to claim 1, wherein
the sensor (110) includes
a base (111) containing an elastomer material, and
an electrically-conductive layer (112a, 112b) that is stacked in the thickness direction of the base (111) and freely expands and contracts integrally with the base (111).

3. The measurement apparatus (100) according to claim 1 or claim 2, wherein
the support member (120, 220) and the sensor (110) are configured to be annularly disposed around the limb in the mounted state.

4. The measurement apparatus (100) according to claim 1 or claim 2, wherein
the support member (120, 220) is configured to be disposed in a spiral manner around the limb in the mounted state.

5. The measurement apparatus (100) according to claim 1, wherein
the adjusting member (130, 230) includes a length detecting part capable of electrically detecting the length of the part that surrounds the limb in the support member (120, 220).

6. The measurement apparatus (100) according to any one of claims 1 to 5, wherein
a plurality of protrusions (123) that protrude toward the limb are disposed on a surface that faces the limb in the support member (120, 220) in the mounted state.

7. The measurement apparatus (100) according to any one of claims 1 to 6, wherein
the support member (120, 220) is composed of a material with a higher modulus of elasticity than the sensor (110).

8. The measurement apparatus (100) according to any one of claims 1 to 7, wherein
a recess part (124) that is formed to hollow in a long axis direction of a leg (D1) in the mounted state and abuts against a projecting part of the limb is made in the support member (120, 220).

## Patentansprüche

1. Messvorrichtung (100), die in der Lage ist, das Ausmaß eines Ödems einer Gliedmaße zu messen,
wobei die Messvorrichtung (100) umfasst:
einen Sensor (110), der eine Blattform aufweist und sich in einer Richtung, die eine Dickenrichtung (Y1) schneidet, frei ausdehnt und zusammenzieht und in der Lage ist, eine Änderung einer elektrischen Eigenschaft in Verbindung mit einer Ausdehnung oder Kontraktion zu erfassen; und
ein Trägerelement, das an beiden Endabschnitten in einer Expansions-Kontraktions-Richtung (X1) des Sensors (110) angebracht ist und die Gliedmaße zusammen mit dem Sensor (110) in einem montierten Zustand umgibt, in welchem die Messvorrichtung (100) an der Gliedmaße angebracht ist,
**dadurch gekennzeichnet, dass**
die Messvorrichtung (100) ferner umfasst:
ein Einstellelement (130, 230), das an dem Trägerelement (120, 220) angeordnet ist und in der Lage ist, die Länge eines Teils, der die Gliedmaße umgibt, in dem Trägerelement (120, 220) in dem montierten Zustand einzustellen; und
ein Begrenzungselement (140), das an dem Trägerelement (120, 220) angebracht werden kann und von dem Trägerelement (120, 220) abnehmbar ist und die Ausdehnung und Kontraktion des Sensors (X1) in einem Zustand, in dem er an dem Trägerelement (120, 220) angebracht ist, einschränkt.

2. Messvorrichtung (100) nach Anspruch 1, wobei
der Sensor (110) umfasst
eine Basis (111), die ein elastomeres Material enthält, und
eine elektrisch leitfähige Schicht (112a, 112b), die in der Dickenrichtung der Basis (111) gestapelt ist und sich zusammen mit der Basis (111) frei ausdehnt und zusammenzieht.

3. Messvorrichtung (100) nach Anspruch 1 oder Anspruch 2, wobei
das Trägerelement (120, 220) und der Sensor (110) so konfiguriert sind, dass sie in dem montierten Zustand ringförmig um die Gliedmaße angeordnet sind.

4. Messvorrichtung (100) nach Anspruch 1 oder Anspruch 2, wobei
das Trägerelement (120, 220) so konfiguriert ist, dass es in dem montierten Zustand spiralförmig rund um die Gliedmaße angeordnet ist.

5. Messvorrichtung (100) nach Anspruch 1, wobei
das Einstellelement (130, 230) ein Längenerfassungsteil umfasst, das in der Lage ist, die Länge des Teils, der die Gliedmaße umgibt, in dem Trägerelement (120, 220) elektrisch zu erfassen.

6. Messvorrichtung (100) nach einem der Ansprüche 1 bis 5, wobei
eine Vielzahl von Vorsprüngen (123), die in Richtung der Gliedmaße vorstehen, auf einer Oberfläche angeordnet sind, die der Gliedmaße in dem Trägerelement (120, 220) in dem montierten Zustand gegenüberliegt.

7. Messvorrichtung (100) nach einem der Ansprüche 1 bis 6, wobei
das Trägerelement (120, 220) aus einem Material mit einem höheren Elastizitätsmodul als der Sensor (110) zusammengesetzt ist.

8. Messvorrichtung (100) nach einem der Ansprüche 1 bis 7, wobei
ein Aussparungsteil (124), welches so ausgebildet ist, dass es in einer Längsachsenrichtung eines Schenkels (D1) in dem montierten Zustand hohl ist und an einem vorstehenden Teil des Schenkels anliegt, in dem Trägerelement (120, 220) ausgebildet ist.

## Revendications

1. Appareil de mesure (100) capable de mesurer une quantité d'œdème dans un membre,
l'appareil de mesure (100) comprenant :
un capteur (110) qui a une forme de feuille et se déploie et se contracte librement dans une direction qui croise une direction d'épaisseur (Y1) et est capable de détecter un changement dans une caractéristique électrique en relation avec le déploiement ou la contraction ; et
un élément de support qui est fixé aux deux portions extrémité dans une direction de déploiement-contraction (X1) du capteur (110) et entoure le membre conjointement au capteur (110) dans un état monté dans lequel l'appareil de mesure (100) est monté sur le membre,
**caractérisé en ce que**
ledit appareil de mesure (100) comprend en outre :
un élément d'ajustement (130, 230) qui est disposé sur l'élément de support (120, 220) et est capable d'ajuster la longueur d'une partie qui entoure le membre dans l'élément de support (120, 220) dans l'état monté ; et
un élément de restriction (140) qui peut être fixé à l'élément de support (120, 220) et peut être détaché de l'élément de support (120, 220) et restreint le déploiement et la contraction du capteur (X1) dans un état dans lequel il est lié à l'élément de support (120, 220).

2. Appareil de mesure (100) selon la revendication 1, dans lequel
le capteur (110) inclut
une base (111) contenant un matériau élastomère, et
une couche électroconductrice (112a, 112b) qui est empilée dans la direction de l'épaisseur de la base (111) et se déploie et se contracte librement en un seul tenant avec la base (111).

3. Appareil de mesure (100) selon la revendication 1 ou la revendication 2, dans lequel
l'élément de support (120, 220) et le capteur (110) sont configurés pour être disposés annulairement autour du membre dans l'état monté.

4. Appareil de mesure (100) selon la revendication 1 ou la revendication 2, dans lequel
l'élément de support (120, 220) est configuré pour être disposé en spirale autour du membre dans l'état monté.

5. Appareil de mesure (100) selon la revendication 1, dans lequel
l'élément d'ajustement (130, 230) inclut une partie de détection de longueur capable de détecter électriquement la longueur de la partie qui entoure le membre dans l'élément de support (120, 220).

6. Appareil de mesure (100) selon l'une quelconque des revendications 1 à 5, dans lequel
une pluralité de saillies (123) qui font saillie en direction du membre sont disposées sur une surface qui fait face au membre dans l'élément de support (120, 220) dans l'état monté.

7. Appareil de mesure (100) selon l'une quelconque des revendications 1 à 6, dans lequel
l'élément de support (120, 220) est composé d'un matériau avec un module d'élasticité supérieur à celui du capteur (110) .

8. Appareil de mesure (100) selon l'une quelconque des revendications 1 à 7, dans lequel
une partie cavité (124) qui est formée pour creuser dans une direction d'axe longitudinal d'une jambe (D1) dans l'état monté et bute contre une partie en saillie du membre est réalisée dans l'élément de support (120, 220).
